(19) Europäisches Patentamt / European Patent Office / Office européen des brevets

(11) **EP 3 643 282 A1**

(12) **EUROPEAN PATENT APPLICATION**
published in accordance with Art. 153(4) EPC

(43) Date of publication:
**29.04.2020 Bulletin 2020/18**

(21) Application number: **18862907.5**

(22) Date of filing: **24.08.2018**

(51) Int Cl.:
**A61F 13/534** *(2006.01)*    **A61F 13/532** *(2006.01)*

(86) International application number:
**PCT/JP2018/031280**

(87) International publication number:
**WO 2019/065023 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2017 JP 2017189905**

(71) Applicant: **Daio Paper Corporation**
**Shikokuchuo-shi, Ehime 799-0492 (JP)**

(72) Inventor: **SUYAMA, Junnosuke**
**Sakura-shi**
**Tochigi 329-1411 (JP)**

(74) Representative: **Williams Powell**
**11 Staple Inn**
**London WC1V 7QH (GB)**

(54) **ABSORBENT ARTICLE**

(57) To further increase the height of a protrusion per unit area of a sheet while maintaining the thinness of the sheet of an absorbent article and absorb more excretion liquid.

An absorbent article according to the present invention has a front surface side sheet 51 and a back surface side sheet 52 disposed on a back surface side of the front surface side sheet 51. A large number of cells 55 are formed by bonding the front surface side sheet 51 and the back surface side sheet 52. A particulate material containing super absorbent polymer particles 53 is accommodated in the cell 55 to form an absorber 50. At least a part of the particulate material containing the super absorbent polymer particles 53 is not fixed to at least one of the front surface side sheet 51 and the back surface side sheet 52. In at least one of the front surface side sheet 51 and the back surface side sheet 52, sites other than the sites where the sheets are bonded each other are shrunk into a pleat shape.

**EP 3 643 282 A1**

**Description**

Technical Field

The present invention relates to an absorbent article such as a disposable diaper and a sanitary napkin.

Background Art

The absorbent article includes an absorber and a liquid-pervious top sheet covering the front surface side of the absorber. Excretion liquid such as urine and menstrual blood passes through the top sheet and is absorbed and held by the absorber. As the absorber, super absorbent polymer particles (SAP) which are mixed with hydrophilic short fibers such as fluff pulp and piled up in a cotton form is widely used. Therefore, Patent Literature 1 proposes "absorbent articles in which, when an absorbent site absorbs body fluid, super absorbent polymer particles expand and a first sheet in the absorbent site protrudes so as to rise upward in the thickness direction, and on the other hand, a second sheet protrudes so as to rise downward in the thickness direction, or increases the thickness without rising, and the dimension of the first sheet protruding upward is larger than the dimension of the second sheet protruding downward". As a result, Patent Literature 1 describes that a moist absorbent site has a small contact area with the skin and hardly gives a moist feeling to the skin, and an air-pervious gap is formed between the skin and a non-absorbent site. That is, Patent Literature 1 describes to ensure high air permeability due to the difference in the protrusion caused by using materials having different stretch rates for the first sheet and the second sheet.

Citation List

Patent Literature

Patent Literature 1: JP 2010-63815 A

Summary of Invention

Technical Problem

However, in the method of Patent Literature 1, since the stretch rate of a sheet itself is determined, the volume in a surrounding portion (cell) is also determined. For this reason, there is a limit to the amount of super absorbent polymer particles that can be accommodated, and when the amount accommodating super absorbent polymer particles exceeds, gel blocking is caused the capacity, or the super absorbent polymer particles flow out from gaps between fibers of the stretched sheet to the outside of the sheet. Therefore, it must be said that there is a limit to the height of the protrusion of the sheet in Patent Literature 1.
Therefore, the problem to be solved by the present invention is to provide an absorbent article that further increases the height of the protrusion per unit area of the sheet while maintaining the thinness of the sheet and absorbs more excretion liquid.

Solution to Problem

Representative means for solving the above problems are as follows.

<Invention according to claim 1>

An absorbent article, including:

a front surface side sheet; and
a back surface side sheet disposed on a back surface side of the front surface side sheet,
wherein a large number of cells are formed by bonding the front surface side sheet and the back surface side sheet,
a particulate material containing super absorbent polymer particles is accommodated in the cell to form an absorber,
at least a part of the particulate material containing the super absorbent polymer particles is not fixed to at least one of the front surface side sheet and the back surface side sheet, and
in at least one of the front surface side sheet and the back surface side sheet, other sites than the sites where the sheets are bonded each other are shrunk into a pleat shape.

(Function and Effect)

The front surface side sheet and the back surface side sheet are bonded to form a large number of cells, and at least one of the front surface side sheet and the back surface side sheet is shrunk into a pleat shape. The super absorbent polymer particles inside the cells are expanded by excretion liquid to stretch pleats of the sheet. As a result, it has the effect that the sheet protrudes in an up-down direction with respect to a plane of the absorber. In particular, in the case where the sheet is a non-woven fabric, when the super absorbent polymer particles are expanded, those are raised to some extent due to the stretch properties of the non-woven fabric without pleats, and are raised more prominently due to pleats. Further, free movement of the particulate material in the cell is limited by having the pleat shape.

<Invention according to claim 2>

The absorbent article according to claim 1, wherein the pleat shape is a wave shape that contracts in a width direction of the absorber.

(Function and Effect)

By disposing vertically-striped plates formed by shrinking in the horizontal direction in the front-back direction of the absorber, there is an effect of blocking the diffusion of excretion liquid in the horizontal direction and the movement of the particulate material to some extent.

<Invention according to claim 3>

The absorbent article according to claim 1 or 2, wherein the pleat shape after shrinking has a shrinkage rate of 15 to 50%.

(Function and Effect)

By arbitrarily selecting the shrinkage rate, it is possible to form absorbers having different heights of protrusion and ease of movement of particulate materials. If the shrinkage rate is less than 15%, the pleat shape does not substantially shrink, such that the effect that the pleat shape extends and the sheet protrudes cannot be obtained. On the other hand, if the shrinkage ratio exceeds 50%, the pleat shape of the sheet becomes too dense, the absorber becomes thick, and the thinness that is an advantage of the absorber cannot be utilized.

<Invention according to claim 4>

The absorbent article according to any one of claims 1 to 3, wherein the cell has a rectangular lattice shape that is long in a width direction of the absorber.

(Function and Effect)

Since the lattice of each cell is rectangular, and the sheet is laterally shrunk, more particulate material can be accommodated in each cell than a square lattice, and the sheet is raised higher. Therefore, it has an effect of absorbing more excretion liquid.

<Invention according to claim 5>

The absorbent article according to claim 4, wherein bonded portions extending in a front-back direction among bonded portions forming the rectangular lattice-shaped cells are weak bonded portions.

(Function and Effect)

When the particulate material in the cell adjacent to the weak bonded portion absorbs excretion liquid and protrudes, the bonded portion is peeled off, and the adjacent cells are united to form a large cell. Since it is obvious that the sheet of the large cell is shrunk, the height of the protrusion of the sheet can be further increased, and the amount of excretion liquid absorbed can be increased. In addition, normal bonded portions (not weak bonded portions) do not protrude, such that the diffusion of excretion liquid is directed.

<Invention according to claim 6>

The absorbent article according to any one of claims 1 to 5, wherein at least one of the front side surface sheet and the back side surface sheet is repeatedly processed into a range in which the shrinkage rate is high and a range in which the shrinkage rate is low.

(Function and Effect)

Since the sheets are processed regularly and repeatedly in the range with high shrinkage and the range with low shrinkage, the height of the protrusion increases in the high range and decreases in the low range. That is, there is an effect that the height of the protrusion is uneven, and the amount of excretion liquid absorbed increases in the high range and decreases in the low range.

<Invention according to claim 7>

The absorbent article according to claim 6, wherein a basis weight of the super absorbent polymer particles is higher in a range where the shrinkage rate is high than in a range where the shrinkage rate is low.

(Function and Effect)

By providing the high and low basis weight of the super absorbent polymer particles, there is an effect that the uneven absorption amount of the excretion liquid can be made remarkable.

Advantageous Effects of Invention

According to the present invention, the absorbent article that can further increase the height of the protrusion per unit area of the sheet and absorb more excretion liquid is achieved.

Brief Description of Drawings

Fig. 1 is a plan view illustrating the inner surface of a tape-type disposable diaper in a state where a diaper is spread.
Fig. 2 is a plan view illustrating the outer surface of a tape-type disposable diaper in a state where a diaper is spread.
Fig. 3 is a cross-sectional view taken along line 6-6 in Fig. 1.
Fig. 4 is a cross-sectional view taken along line 7-7 in Fig. 1.
Fig. 5(a) is a cross-sectional view taken along line 8-8 in Fig. 1, and Fig. 5(b) is a cross-sectional view taken along line 9-9 in Fig. 1.
Fig. 6 is a cross-sectional view taken along line 5-5 in Fig. 1.
Fig. 7 is a processing example of a front surface side sheet and a back surface side sheet.
Fig. 8 is a conceptual diagram of wave shape processing of a front surface side sheet and a back surface side sheet.
Fig. 9 is a plan view of an embodiment of a bonding pattern of a bonded portion and a cross-sectional view taken along line 4-4 in Fig. 9.
Fig. 10 is a plan view of an embodiment of a bonding pattern of a bonded portion.
Fig. 11 is a cross-sectional view of an absorber with a shrinkage rate increased or decreased.
Fig. 12 is a cross-sectional view of an absorber before and after expansion.

Description of Embodiments

Hereinafter, embodiments of the present invention will be described.
Conventionally, the use of absorbent articles varies widely, such as disposable diapers and sanitary napkins. Among these, a form in the case of wearing absorbent articles inside disposable diapers will be explained in full detail. Note that the following description and drawings merely show one embodiment of the present invention.
As illustrated in Figs. 1 to 7, an absorbent article according to the present invention has a front surface side sheet 51 and a back surface side sheet 52 disposed on a back surface side of the front surface side sheet 51. A large number of cells 55 are formed by bonding the front surface side sheet 51 and the back surface side sheet 52. A particulate material containing super absorbent polymer particles 53 is accommodated in the cell 55 to form an absorber 50. At least a part of the particulate material containing the super absorbent polymer particles 53 is not fixed to at least one of the front surface side sheet 51 and the back surface side sheet 52. In at least one of the front surface side sheet and the back surface side sheet, sites other than the sites where the sheets are bonded each other are shrunk into a pleat shape. In addition, the pleat shape is preferably one that is shrink-processed in the width direction WD of the absorber 50.

(Pleat shape)

The pleats of the front surface side sheet 51 and the back surface side sheet 52 (hereinafter referred to simply as "sheets 51 and 52" when the front surface side and the back surface side are not specifically described) refers to a surface where protrusions and recesses are formed regularly and repeatedly on the sheets 51 and 52. As the material of the sheets 51 and 52, a liquid-pervious material, a liquid-impervious material, a non-woven fabric, or the like can be used as described later. For example, the nonwoven fabric is regularly folded and pressurized to form wave formed pleats. Further, wave formed or uneven shape pleats are formed by pressurizing with an uneven roll. The processed pleat-shaped nonwoven fabric maintains its shape and does not naturally return to the state without pleats before processing.

The interval between the pleats (one cycle) is preferably 3 to 25 mm, and the amplitude of the pleats is preferably 1 to 25 mm.

When force is applied to the sheets 51 and 52 processed into a pleat shape in the direction of extending pleats, the pleats easily extend. Therefore, when the sheets 51 and 52 processed into a pleat shape containing the super absorbent polymer particles 53 are absorbed with liquid, due to the expansion of the super absorbent polymer particles 53, the pleats of the sheets 51 and 52 also extend at least by a shrinkage rate (definition of the shrinkage rate will be described later). Note that, the nonwoven fabric itself has a certain degree of extensibility.

As illustrated in Fig. 7, examples of the pleat type processing of the sheets 51 and 52 include wave processing, uneven processing, deformation uneven processing with the bottom of a recess and protrusion widened, or processing into a locus shape in which semicircular arcs are continuously arranged on a zigzag line.

Furthermore, the pleat processing can be performed in a planar manner. For example, there is a process in which the protrusion shape spreads in a plane like many lattice points.

However, it is not limited to these processes. It is sufficient that the pleat type of the sheets 51 and 52 is processed such that pleats spread by the expansion of the super absorbent polymer particles 53, and the area of the sheets 51 and 52 of the cell 55 when the cell 55 protrudes is larger than the area of the cell 55 before the protrusion.

In principle, the absorbent article is composed of the front surface side sheet 51, the back surface side sheet 52, and the super absorbent polymer particles 53 formed by these processes, and new sheet or the like is not required separately, and therefore the conventional thinness can be maintained.

(Direction of pleat)

As illustrated in Fig. 8, as for the pleats direction, taking the case of performing wave processing on the sheets 51 and 52 as an example, on the surfaces of the sheets 51 and 52, a large number of straight lines of mountain folds are formed in parallel, and on the back surface, the same number of straight lines of valley folds are formed in parallel, that is formed in a stripe type. The linear direction of the mountain fold may be arranged parallel to the front-back direction LD of the absorber 50 or may be arranged perpendicularly. However, for the sake of convenience, the description will be made assuming that the linear direction is arranged in parallel unless otherwise specified.

(Shrinkage)

The processed sheets 51 and 52 shrink compared to the unprocessed sheets 51 and 52. Here, a shrinkage rate is defined as follows.

$$\text{Shrinkage rate } (\%) = (\text{area of unprocessed sheets 51 and 52} - \text{area of processed sheets 51 and 52})/(\text{area of unprocessed sheets 51 and 52}) \times 100$$

The above-described stretch rate refers to an elongation with respect to the original sheet length when the sheet is stretched in opposite directions from both sides of the sheet, and is a concept different from the shrinkage rate here.

From the viewpoint that it is better to absorb more excretion liquid, it is preferable to increase the shrinkage rate and contain a large amount of the super absorbent polymer particles 53. However, if the shrinkage rate is higher than necessary, in the initial state, that is, before the product is used, folds of the pleats are dense, and therefore the sheets 51 and 52 are bulky, and the absorber 50 becomes thick. As a result, the thinness that is an advantage of the absorber 50 is not utilized, which is inconvenient. On the other hand, if the shrinkage rate is lower than a predetermined value, the effect that the pleat shape extends and the sheets 51 and 52 protrude cannot be sufficiently obtained. Therefore, the shrinkage rate is, for example, 15 to 50%, more preferably 35%.

(Shape of cell 55)

The cell 55 refers to a space having a partitioned constant volume formed by bonding the front surface side sheet 51 and the back surface side sheet 52 disposed on the back surface side of the front surface side sheet 51. For example, when the square cell 55 is formed, portions on the corresponding sides of the squares in the front surface side sheet 51 and the back surface side sheet 52 disposed thereon may be bonded.

The super absorbent polymer particles 53 are accommodated in the cells 55 in principle. However, in order to prevent the super absorbent polymer particles 53 existing in the vicinity of the edge of the front surface side sheet 51 and the back surface side sheet 52 from flowing out to the outside, it is preferable to extend an edge bonded portion over the entire edge of the front surface side sheet 51 and the back surface side sheet 52.

Since the sheets 51 and 52 of the cell 55 are shrink-processed into a pleat shape, when the super absorbent polymer particles 53 in the cell 55 absorb excretion liquid, the pleat shape extends due to the expansion of the super absorbent polymer particles 53. As a result, the sheets 51 and 52 of the cell 55 expand in a balloon shape.

(Bonded portion pattern)

As illustrated in Figs. 9 and 10, the pattern of the bonded portion may be as follows. Note that Fig. 9 exemplifies the case where the front surface side sheet 51 expands, but it is obvious that the case where the back surface side sheet 52 expands or the case where both the front surface side sheet 51 and the back surface side sheet 52 expand are also possible forms.

A lattice-like bonded portion can be disposed in pattern 1. More preferably, a bonded portion 54 having a rectangular lattice shape can be disposed along the front-back direction LD of the absorber 50. The length aa in the width direction WD of each cell 55 partitioned in a lattice shape is longer than the length bb in the front-back direction LD. For example, the size of the cell 55 is preferably about 8 to 30 mm in length and about 10 to 50 mm in width. In this way, since the direction of the pleat-shaped mountain fold is parallel to the front-back direction LD, the sheets 51 and 52 extend in the width direction WD and become bulky when the sheets are raised, such that excretion liquid can be absorbed more than before. In addition, when the sheets bulky protrudes, the contact area with the wearer's skin is reduced, and the uncomfortable feeling during wearing is reduced. Furthermore, as described below, a part of the super absorbent polymer particles 53 can be non-adhered to at least the sheets 51 and 52. In this case, the super absorbent polymer particles 53 can move freely in the cell 55, but by making the sheets 51 and 52 into a pleat shape, the super absorbent polymer particles are caught by the pleats, the free movement is restricted to some extent, and there is an effect of preventing uneven distribution of the super absorbent polymer particles 53.

In pattern 2, as illustrated in the upper left of Fig. 10, first, a rectangular lattice-like bonded portion 54 is disposed as in pattern 1 above, and then two adjacent cells 55 located at the center in the width direction WD are specified. Among the rows of the cells 55 extending in the front-back direction LD from the two cells 55, a group of the cells 55 positioned in a zigzag manner is further specified. For each of a group of the cells 55, the bonded portion 54 is provided so as to be equally divided into four rectangular sections. In this way, since there is a difference in the bulkiness of the protrusions in the two central rows of the absorber 50 in the width direction WD when the sheets protrude, the direction of excretion liquid diffusion varies.

In pattern 3, the direction of the lattice-like bonded portion 54 and the pleat shape is arranged as in the above pattern 1. However, the bonding strength of the bonded portion in the front-back direction LD is weakened. For example, the size of the cell 55 is preferably about 8 to 30 mm in length and about 10 to 50 mm in width (distance between the weak bonded portions 54b). As a result, the weak bonded portion 54b is peeled off due to the expansion of the super absorbent polymer particles 53. The sheets 51 and 52 have a substantially semi-cylindrical shape that protrudes and extends in the width direction WD. Since a plurality of the substantially half-cylinder-shaped extensions is formed in parallel in the front-back direction LD, it is difficult for further excretory liquid to diffuse in the front-back direction LD. Further, since there is room for the weak bonded portion 54b to protrude as compared with pattern 1, a large amount of excretion liquid can be absorbed.

In pattern 4, from the end in the width direction WD to the other end, the bonded portion 54 is formed in a wave shape, and a plurality of the wave shapes is disposed in the front-back direction LD. By disposing a plurality of wave shapes densely, a substantially closed space (cell 55) is formed between the wave shapes. The size of the wave shape is preferably, for example, a cycle length of 20 to 100 mm and an amplitude of about 8 to 30 mm. In this way, since the bonded portion 54 is not a straight line, the excretion liquid does not diffuse linearly, and the diffusion of the excretion liquid to the end of the absorber 50 is delayed, and there is an effect of preventing liquid leakage.

Pattern 5 may have a rhombus shape on the plane without gaps, that is, an oblique lattice shape. The length of the diagonal line of the rhomboid cell 55 is preferably about 8 to 30 mm in length and about 10 to 50 mm in width, for example. In this way, the diffusion of the excretion liquid spreads in an oblique direction and the excretion liquid can be prevented from directly diffusing in the width direction WD or the front-back direction LD.

(Protrusion)

Next, the expansion of the super absorbent polymer particles 53 will be described.

When the excretion liquid passes through the sheets 51 and 52 and is absorbed by the super absorbent polymer particles 53 in the cell 55, the super absorbent polymer particles 53 expand to a predetermined volume. Adjacent super absorbent polymer particles 53 also expand due to absorption of excretion liquid. Thus, the adjacent super absorbent polymer particles 53 expand one after another, and try to spread the pleat shape formed on the sheets 51 and 52 of the cell 55. As a result, the pleat shape of the sheets 51 and 52 that are initially deflated before absorbing the excretion liquid spreads, expands and protrudes by the expansion force of the super absorbent polymer particles 53 inside. Note that Fig. 12 exemplifies the case where the front surface side sheet 51 expands, but it is obvious that the case where the back surface side sheet 52 expands or the case where both the front surface side sheet 51 and the back surface side sheet 52 expand are also possible forms.

In Patent Literature 1 described above, the sheet protrudes due to absorption of the super absorbent polymer particles 53, but this is due to an elongation rate of the sheet itself. On the other hand, the protrusion in the present invention is not due to the elongation rate of the sheet itself, but it is due to the spread of the preprocessed pleat shape, and the protruding means are completely different. Further, the present invention has an advantage that the degree of the bulkiness of the protrusion can be adjusted according to the objective of an absorbent article by changing a shrinkage rate arbitrarily.

(High and low shrinkage ratio)

As the sheets 51 and 52, sheets having a difference in level of shrinkage rate may be used. That is, it is possible to use a material in which a range where the shrinkage rate is high and a range where the shrinkage rate is low are regularly repeatedly processed. For example, in wave processing, it is possible to perform processing such that a portion having a shrinkage rate of 30% (high shrinkage rate) and a portion having a shrinkage rate of 15% (low shrinkage rate) are alternately repeated.

(Example with height difference)

As illustrated in Fig. 11, for the lattice-like cell 55 of pattern 1 described above, if there is a difference in the shrinkage rate, it is possible to form the absorber 50 in which the cell 55 rows formed with a high shrinkage rate and the cell 55 rows formed with a low shrinkage rate are alternately repeated in the width direction WD. When the absorber 50 absorbs the excretion liquid, the cells 55 that protrude high and the cells 55 that protrude low are alternately formed in the width direction WD, such that the effect of preventing excretion liquid from spreading in the width direction WD can be expected.

Further, the shrinkage rate of the sheets 51 and 52 is assumed to be higher in the cell 55 rows near the center of the width direction WD, and lower in the cell 55 rows at the left and right ends. As a result of absorption of the excretion liquid, in the absorber 50, the cell 55 rows in the vicinity of the center protrude higher than the cell 55 rows at the end. This absorber 50 can cope with a case where even if the amount of excretion liquid at one time is particularly small, excretion is performed multiple times.

Further, to make the difference in the height of the protrusion noticeable, a difference in the basis weight of the super absorbent polymer particles 53 may be provided. It is preferable that the basis weight of the super absorbent polymer particles 53 in the cell 55 formed with a high shrinkage is 200 to 350 $g/m^2$, and the basis weight in the cell 55 formed with a low shrinkage is 50 to 200 $g/m^2$. Note that Fig. 11 exemplifies the case where the front surface side sheet 51 expands, but it is obvious that the case where the back surface side sheet 52 expands or the case where both the front surface side sheet 51 and the back surface side sheet 52 expand are also possible forms.

<Example of Absorbent Article>

Figs. 1 to 6 illustrate examples of a tape-type disposable diaper, in which the reference sign X indicates the entire width of the diaper excluding a fastening tape, and the reference sign L indicates the entire length of the diaper. Each component member is fixed or bonded in the same manner as known diapers as necessary except for the fixed or bonded portion described below. As a unit for fixing or bonding, a hot melt adhesive or welding (heat welding, ultrasonic welding) can be selected as appropriate.

This tape type disposable diaper has a basic structure in which an absorber 50 is interposed between a liquid-pervious top sheet and a liquid-impervious sheet 11 located on the external surface side, and includes a ventral side end flap portion EF, a dorsal side end frap portion EF, and a pair of side flap portion SF. The ventral side end flap portion EF and the dorsal side end flap portion EF are portions extending to the front side and the back side of the absorber 50, respectively, that are portions where the absorber 50 are not included. A pair of the side flap portion SF extends laterally from the side edge of the absorber 50. In each of the side flap portions SF in a dorsal side portion B, a fastening tape 13 is provided. When a user wears the diaper, the fastening tape 13 is engaged at an appropriate place on the external surface of the ventral side portion F in a state in which the side flap portion SF of the dorsal side portion B is piled on the external side of the side flap portion SF of the ventral side portion F.

In this tape type disposable diaper, the entire external surface of the absorbent main unit 10 and the side flap portions SF is formed by an outer sheet 12. Particularly, in a region including the absorber 50, a liquid-impervious sheet 11 is fixed to the internal surface side of the outer sheet 12 with an adhesive such as a hot melt adhesive. Further, the absorber 50, an intermediate sheet 40, and a top sheet 30 are stacked in this order on the internal surface side of the liquid-impervious sheet 11. In the illustrated example, the top sheet 30 and the liquid-impervious sheet 11 are rectangular in shape and have somewhat larger sizes in the front-back direction LD and the width direction WD than the absorber 50. The peripheral edges protruding from the side edges of the absorber 50 in the top sheet 30 and the peripheral edges protruding from the side edge of the absorber 50 in the liquid-impervious sheet 11 are bonded by a hot melt adhesive or the like. Further, the liquid-impervious sheet 11 is formed to be slightly wider than the top sheet 30.

On both sides of the absorbent main unit 10, three-dimensional side gathers 60 projecting (standing) to the skin side of a wearer are provided, and gather sheets 62 forming the three-dimensional side gathers 60 are fixed in a range from the upper both sides of the top sheet 30 to the inner surface of each of the side flap portions SF.

Details of each part will be described in order below.

(Outer Sheet)

The outer sheet 12 is a sheet constituting the external surface of a product. The outer sheet 12 has a shape in which the central portions of the front-back direction LD on the both side portions are narrowed, and these portions surround the wearer's legs. A nonwoven fabric is suitable for the outer sheet 12, but it is not limited thereto. The type of the nonwoven fabric is not particularly limited. As a raw material fiber, for example, in addition to synthetic fibers such as polyolefin type such as polyethylene or polypropylene, polyester type, and polyamide type, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spunbonded nonwoven fabric, an SMS nonwoven fabric, and an SMMS nonwoven fabric are preferable in that both good touch feeling and strength can be compatible. In addition to using a single piece of nonwoven fabric, it is also possible to use multiple nonwoven fabrics in layers. In the latter case, it is preferable that the nonwoven fabrics are adhered to each other with a hot melt adhesive or the like. When a nonwoven fabric is used, the basis weight of the fiber is desirably 10 to 50 $g/m^2$, particularly desirably 15 to 30 $g/m^2$. The outer sheet 12 can be omitted, and in that case, the liquid-impervious sheet 11 can have the same shape as that of the outer sheet 12, such that the outer surface of a product can be formed.

(Liquid-impervious Sheet)

Although the material of the liquid-impervious sheet 11 is not particularly limited, for example, a polyolefin-based resin such as polyethylene or polypropylene, a laminated nonwoven fabric obtained by stacking a nonwoven fabric on a polyethylene sheet or the like, a nonwoven fabric in which liquid permeability is substantially secured through a water proof film (in this case, a liquid-impervious sheet is formed by the waterproof film and the nonwoven fabric) can be exemplified. Obviously, besides this, in recent years, liquid-impervious and moisture-pervious materials which have been favorably used from the standpoint of prevention of stuffiness can also be exemplified. As a sheet of this liquid-impervious and moisture-pervious material, for example, a microporous sheet can be exemplified which is obtained by stretching a sheet in one or two axial directions, the sheet being molded after kneading an inorganic filler in a polyolefin-based resin such as polyethylene or polypropylene. Further, nonwoven fabrics using micro denier fibers and a sheet that is liquid-impervious without using a water proof film can also be used as the liquid-impervious sheet 11. The sheet has a high leak proof obtained by heating or applying pressure to reduce air gaps of fibers, and a liquid impermeability obtained by applying a super absorbent resin, a hydrophobic resin, or a water repellent agent.

(Top Sheet)

As the top sheet 30, a porous or non-porous nonwoven fabric having liquid permeability can be used. The type of constituent fibers of the nonwoven fabric is not particularly limited. Examples of the nonwoven fabric can include synthetic fibers such as polyolefin type such as polyethylene and polypropylene, polyester type, and polyamide type, regenerated fibers such as rayon and cupra, natural fibers such as cotton, mixed fibers in which two or more of these are used, and composite fibers. Further, the nonwoven fabric may be manufactured by any processing. Examples of the processing method include known methods such as a spun lace method, a spun bond method, a thermal bond method, a meltblown method, a needle punch method, an air-through method, and a point bond method. For example, the spun lace method is preferable when flexibility and drapability are required, and the thermal bonding method is preferable when bulkiness and softness are required.

(Intermediate Sheet)

The intermediate sheet 40 is bonded to the back surface of the top sheet 30 to promptly move excretion liquid passing through the top sheet 30 to the side of the absorber 50 and to prevent backflow. For bonding between the intermediate sheet 40 and the top sheet 30, heat embossing or ultrasonic welding can be used in addition to using a hot melt adhesive. As the intermediate sheet 40, a resin film having a large number of through-holes can be used in addition to using a nonwoven fabric. As the nonwoven fabric, a material similar to that described in the section of the top sheet 30 can be used. However, the material having a higher hydrophilicity than that of the top sheet 30 or the material having a high fiber density is preferable since those have excellent liquid transfer characteristics from the top sheet 30 to the intermediate sheet 40.
Although the intermediate sheet 40 in the illustrated embodiment is shorter than the width of the absorber 50 and disposed at the center, it may be provided throughout the entire width. The length of the intermediate sheet 40 in the front-back direction LD may be the same as the entire length of a diaper, may be the same as the length of the absorber 50, or may be within a short length range around a region receiving a liquid.

(Three-dimensional Side Gather)

To prevent lateral movement of excrement on the top sheet 30 and to prevent lateral leakage, it is preferable to provide the three-dimensional side gather 60 projecting (standing) from the inner faces on both sides of the product in the width direction WD.
The three-dimensional side gather 60 is composed of a gather sheet 62 and an elongated elastically stretchable member 63 fixed to the gather sheet 62 in a stretched state along the front-back direction LD. As this gather sheet 62, a water repellent nonwoven fabric can be used, and rubber thread and the like can be used as the elastically stretchable member 63. As illustrated in Figs. 1 and 3, one or a plurality of the elastically stretchable members 63 can be provided for each.
The inner surface of the gather sheet 62 has a fixed start point in the width direction WD on the side portion of the top sheet 30. A portion outside the width direction WD from this fixed start point is fixed with a hot melt adhesive or the like on the side portion of the liquid-impervious sheet 11 and the side portion of the outer sheet 12 positioned at the outside portion.
In the periphery of the legs, the inside of the width direction WD from the fixed start point of the three-dimensional side gather 60 is fixed on the top sheet 30 at both ends of the product front-back direction LD. However, the portion therebetween is a non-fixed free portion erected by contraction force of the elastically stretchable member 63. Since the diaper is attached to the body in a boat shape at the time of wearing the diaper, and the contraction force of the elastically stretchable member 63 acts, the three-dimensional side gathers 60 erect by the contraction force of the elastically stretchable member 63 and come in close contact with the legs. As a result, lateral leakage from around the legs is prevented.
Unlike the illustrated embodiment, both end portions of the front-back direction LD in the portion of the inside of the width direction WD of the gather sheet 62 are fixed in a state folded in two having a base end side portion extending inward from a portion outside the width direction WD and a tip side portion folded back on the body side from the end edge of the center side in the width direction WD of the base end side portion and extending to the outside in the width direction WD, and the portion therebetween may be a non-fixed free portion.

(Flat Gather)

As illustrated in Figs. 1 to 3, in each side flap portion SF, on the outside in the width direction WD in the vicinity of the fixed start point of the fixed portion of the gather sheet 62, between the gather sheet 62 and the liquid-impervious sheet 11, the elastically stretchable members 64, which are made of rubber thread and the like, around the leg portions are fixed in a state stretching along the front-back direction LD, whereby the leg portion of each side flap portion SF is formed as a flat gather. The elastically stretchable members 64 around the leg portions can also be disposed between the liquid-impervious sheet 11 and the outer sheet 12 in the side flap portion SF. As in the illustrated example, a plurality of elastically stretchable members 64 around the leg portions may be provided on each side, or only one elastically stretchable member 64 may be provided on each side.

(Fastening Tape)

As illustrated in Figs. 1, 2, and 6, the fastening tape 13 includes a sheet base material forming a tape attaching portion 13C fixed to the side portion of a diaper and a tape main unit section 13B projecting from the tape attaching portion 13C, and an engagement portion 13A with respect to the ventral side, which is provided in the middle of the width direction WD of the tape main unit section 13B in the seat base material. A tip end side from the engagement portion 13A is a tab part. The tape attaching portion 13C of the fastening tape 13 is sandwiched between the gather sheet 62 forming the inner layer in the side flap portion and the outer sheet 12 forming the outer layer and is adhered to the both sheets 62 and 12 with the hot melt adhesive. In addition, the engagement portion 13A is unpeelably bonded to the sheet base material with an adhesive.

A hook member (male member) of a mechanical fastener (hook and loop fastener) is suitable as the engagement portion 13A. The hook member has a large number of engagement projections on its outer surface side. The engagement projection has a check mark shape, a J shape, a mushroom shape, a T shape, and a double J shape (a shape bonded back to back of a J shape), but may have any shape. Obviously, an adhesive material layer can also be provided as an engagement portion of the fastening tape 13.

As the sheet base material forming from the tape attaching portion to the tape main unit section, in addition to various nonwoven fabrics such as a spunbonded nonwoven fabric, an air-through nonwoven fabric, and a spunlace nonwoven fabric, a plastic film, a polyethylene laminated nonwoven fabric, paper, or a composite material thereof can be used.

(Target Sheet)

It is preferable to provide a target sheet 12T having a target for facilitating engagement at the engagement portion of the fastening tape 13 in the ventral side portion F. In the case where the engagement portion is the hook member 13A, the target sheet 12T can be used in which a large number of loop threads to which an engagement projection of a hook member is tangled are provided on a surface of the sheet base member made of a plastic film or a nonwoven fabric. Further, in the case of an adhesive layer, it is possible to use a sheet base material made of a plastic film having a smooth surface with high adhesiveness and subjected to peel treatment. In the case where the engagement point of the fastening tape 13 in the ventral side portion F is made of a nonwoven fabric, for example, when the outer sheet 12 in the illustrated embodiment is made of a nonwoven fabric, and the engagement portion of the fastening tape 13 is the hook member 13A, the target sheet 12T may be omitted, and the hook member 13A can be entangled and engaged with the nonwoven fabric of the outer sheet 12. In this case, the target sheet 12T may be provided between the outer sheet 12 and the liquid-impervious sheet 11.

(Absorber)

The absorber 50 is a part that absorbs and retains the liquid content of excrement. The absorber 50 includes the front surface side sheet 51, the back surface side sheet 52 disposed on the back side of the front surface side sheet 51, and the super absorbent polymer particles 53. Not to fall off from the absorber, the super absorbent polymer particles 53 are sandwiched between the front surface side sheet 51 and the back surface side sheet 52 and accommodated in the cell 55 formed by bonding the periphery thereof. In this way, the absorber 50 can have a large number of cells 55. Further, a middle sheet 80 (not illustrated) can be disposed between the front surface side sheet 51 and the back surface side sheet 52.

The absorber 50 can be adhered (bonded) to a member on at least one side of its front and back surfaces via an adhesive such as a hot melt adhesive. The bonded portions 54 of the cells 55 are bonded in a uniform pattern, and the front surface side sheet 51 and the back surface side sheet 52 are formed by the above-described pleat processing. By distributing and retaining the super absorbent polymer particles 53 in a large number of cells 55 surrounded by the bonded portion 54, the uneven distribution of the super absorbent polymer particles 53 in the absorber 50 can be prevented.

Further, the absorber 50 can be wrapped with a wrapping sheet (not illustrated). In this case, one wrapping sheet can be wrapped in a cylindrical shape so as to surround the front and back surfaces and both side surfaces of the absorber 50 and also wrapped so as to sandwich from both the front side and the back side by two wrapping sheets. As the wrapping sheet, a tissue paper, particularly a crepe paper, a nonwoven fabric, a polyethylene laminated nonwoven fabric, a sheet with small holes, and the like can be used. However, it is desirable that the sheet from which the super absorbent polymer particles 53 do not come off is used. When a nonwoven fabric is used for a wrapping sheet, a hydrophilic SMS nonwoven fabric (SMS, SSMMS, etc.) is particularly suitable, and polypropylene and polyethylene/polypropylene composite material can be used as a material. The basis weight is desirably 5 to 40 g/m², particularly desirably 10 to 30 g/m². When the absorber 50 is wrapped with a wrapping sheet, pulp fibers can be accumulated on one side of the front and back sides of the absorber 50, and these can be wrapped with the wrapping sheet collectively.

The front surface side sheet 51 is a material for forming the absorber 50, and is used as a pair with the back surface side sheet 52 to hold the super absorbent polymer particles 53 in the absorber 50. The front surface side sheet 51 may be formed of a liquid-pervious material or a liquid-impervious material, but preferably it is formed of a liquid-pervious material when it is positioned on the top sheet 30 side as indicated in the illustrated embodiment. Similarly to the top sheet 30, a porous or non-porous nonwoven fabric or a porous plastic sheet can be used for the front surface side sheet 51. In the case of using a nonwoven fabric for the front surface side sheet 51, examples of the constituent fibers include synthetic fibers (including not only single component fibers but also conjugate fibers) such as polyolefin type such as polyethylene or polypropylene, polyester type, and polyamide type, regenerated fibers such as rayon and cupra, and natural fibers such as cotton, but it can be selected without limitation, and it is preferable to use thermoplastic resin fibers because of excellent thermal processability. The fiber bonding method of the nonwoven fabric is not particularly limited, but to prevent the super absorbent polymer particles 53 from falling off, it is preferable to use a bonding method which increases fiber density, such as a spun bond method, a meltblown method, and a needle punch method. In the case of using a porous plastic sheet, its pore diameter is preferably smaller than the outer shape of the super absorbent polymer particle 53 to prevent the super absorbent polymer particle 53 from falling off. When the material of the front surface side sheet 51 is hydrophobic, a hydrophilic agent can also be contained.

To facilitate the arrangement of the super absorbent polymer particles 53 at the time of manufacture and to secure the volume after absorption expansion, in the portion forming each cell 55 in the front surface side sheet 51, recesses recessed from the back surface side to the front surface side are preferably formed, but it may not be formed.

The back surface side sheet 52 is used as a pair with the front surface side sheet 51, and holds the super absorbent polymer particles 53 in the absorber 50. The back surface side sheet 52 may be made of the same material as the front surface side sheet 51, but in the case where the front surface side sheet 51 is formed of a liquid-pervious material, a liquid-impervious material can be used for the back surface side sheet 52. The liquid-impervious material usable for the back surface side sheet 52 can be appropriately selected and used from the materials described in the section of the liquid-impervious sheet 11. Although not illustrated, the front surface side sheet 51 and the back surface side sheet 52 may be one side layer and another side layer in which one sheet of material is folded in two.

Further, the middle sheet 80 can be made of the same material as the front surface side sheet 51, and may be a liquid-pervious material or a liquid-impervious material. Although not illustrated, the front surface side sheet 51, the middle side sheet, and the back surface side sheet 52 can be formed by three layers obtained by folding a single material into three.

In principle, all of the super absorbent polymer particles 53 are not fixed to the front surface side sheet 51 and the back surface side sheet 52, and are freely movable. However, not all the super absorbent polymer particles 53 are necessarily fixed, and a part of the particles 53 (for example, 0 to 50% by mass, preferably 0 to 30% by mass, more preferably 0 to 10% by mass of the total amount of the particles 53) may be adhered or stuck to the front surface side sheet 51 and the back surface side sheet 52. Further, the super absorbent polymer particles 53 may be agglomerated to some extent.

As the super absorbent polymer particles 53, those used for this type of absorbent articles can be used on an as-is basis. The particle diameter of the super absorbent polymer particles 53 is not particularly limited, but for example, when the particles are sieved (shaking for five minutes) using a standard sieve (JIS Z8801-1:2006) of 500 μm and the resultant minus serve particles are further sieved (shaking for five minutes) using the standard sieve (JIS Z8801-1: 2006) of 180 μm, it is desirable that the proportion of the particles remaining on the 500 μm standard sieve is 30% by weight or less, and the proportion of the particles remaining on the 180 μm standard sieve is 60% by weight or more.

The material of the super absorbent polymer particles 53 can be used without particular limitation, but the material having the amount of water absorption of 40 g/g or more is suitable. Examples of the super absorbent polymer particles 53 include starch-based, cellulose-based, and synthetic polymer-based particles, and starch-acrylic acid (salt) graft copolymers, saponified starch-acrylonitrile copolymers, crosslinked sodiumcarboxymethylcellulose, and acrylic acid (salt) polymers can be used. As the shape of the super absorbent polymer particles 53, the shape of powder particles which are usually used is suitable, but other shapes can also be used.

The super absorbent polymer particles 53 having a water absorption rate of 70 seconds or less, particularly 40 seconds or less, are suitably used. If the water absorption rate is too slow, back-flow, in which the liquid fed into the absorber 50 returns to the outside of the absorber 50, is likely to occur.

The super absorbent polymer particles 53 having the gel strength of 1,000 Pa or more are preferably used. Thereby, even when the absorber 50 is bulky, it is possible to effectively suppress stickiness after liquid absorption.

The basis weight of the super absorbent polymer particles 53 can be appropriately determined according to the absorption amount required for the use of the absorber 50. Therefore, although it cannot be said unconditionally, the basis weight can be 50 to 350 g/m$^2$. When the basis weight is less than 50 g/m$^2$, it is difficult to ensure the absorption amount. When it exceeds 350 g/m$^2$, the effect is saturated.

The bonded portion 54 that bonds the front surface side sheet 51 and the back surface side sheet 52 is provided in a uniform arrangement throughout the absorber 50, but may be partially changed. It is desirable that the bonded portion 54 be bonded by welding the front surface side sheet 51 and the back surface side sheet 52 like ultrasonic welding or heat sealing, but it may be bonded with a hot melt adhesive.

The bonded portions 54 of the front surface side sheet 51 and the back surface side sheet 52 are arranged so as to surround each cell 55, and as long as it becomes the boundary between adjacent cells 55, the bonded portions 54 may be formed in a continuous line shape as illustrated in the figure, or may be formed in a dotted line shape (intermittently around each cell 55). In the case of intermittently forming the bonded portion 54, the super absorbent polymer particles 53 are not present between the bonded portions 54 in the direction surrounding the cell 55, or even if the super absorbent polymer particles 53 are present between them, less super absorbent polymer particles than those in the cell 55 are included.

In principle, the bonded portion 54 does not peel off due to the expansion force of the super absorbent polymer particles 53 in the adjacent cells 55. However, the weak bonded portion 54b can be peeled off by the same expansion force. This is because part of the super absorbent polymer particles 53 is not fixed to at least the front surface side sheet 51 or the back surface side sheet 52, can be freely moved in a range sandwiched between these sheets 51 and 52, and may fall off the end of the sheets 51 and 52. Not to fall off, the cell 55 bonded at the periphery is provided, and the super absorbent polymer particles 53 are retained in the cell 55. On the other hand, if the excretion liquid is absorbed, although the super absorbent polymer particles 53 expand, there is a limit to the volume of one cell 55 after expansion, and therefore gel blocking is caused, and a sufficient amount of excretion liquid may not be absorbed. Therefore, by allowing the bonded portions to be easily peeled off by the expansion force of the super absorbent polymer particles 53, the adjacent cells 55 are joined together such that more excretion liquid can be absorbed. Therefore, it is preferable to provide the weak bonded portion 54b.

On the other hand, when the bonded portion 54 positioned on the outermost side in the width direction WD is peeled off, there is a possibility that the super absorbent polymer particles 53 or gelled material thereof leaks out to the side of the absorber 50, and therefore it is desirable to increase the strength of the bonded portion 54. From the same viewpoint, it is preferable that the front surface side sheet 51 and the back surface side sheet 52 are extended to the outside in the width direction WD to some extent from the cell 55 forming region, and the edge bonded portion 54c is provided in the extending portion for reinforcement.

The difference of the bonding strength of the bonded portion 54 (strength against peeling of the front surface side sheet 51 and the back surface side sheet 52) may be easily formed by changing the area of the bonded portion 54 but is not limited thereto. For example, in the case of forming the bonded portion 54 with a hot melt adhesive, a method in which the type of a hot melt adhesive is varied depending on the site can be adopted. In particular, when the bonded portion 54 is formed by welding the front surface side sheet 51 and the back surface side sheet 52, the weak bonded portion 54b can also be formed by making the bonded portion a dot shape.

In order to make the weak bonded portion 54b peelable, the volume at the time of saturated absorption of the super absorbent polymer particles 53 in the cell 55 is sufficiently larger than the volume of the cell 55 adjacent to the weak bonded portion 54b. The type and amount of super absorbent polymer particles 53 disposed in each cell 55 can be determined.

In the above example, only the super absorbent polymer particles 53 are contained in the cell 55, but it is also possible to contain the super absorbent polymer particles 53 together with powder particles other than the super absorbent polymer particles 53, such as deodorant particles.

<Explanation of Terms Used Herein>

In the case where the following terms are used in the specification, those have the following meanings unless otherwise specified in the specification.

"Length direction" and "width direction" mean the flow direction (LD) in a manufacturing equipment and the lateral direction (WD) orthogonal to the flow direction, and either one is the front-back direction of a product, and the other is the width direction of the product. The LD of a nonwoven fabric is the direction of fiber orientation of the nonwoven fabric. "Fiber orientation" is a direction along which a fiber of a nonwoven fabric runs and determined by, for example, a measurement method in accordance with the fiber orientation test method based on the zero distance tensile strength of TAPPI standard method T481 and a simple measurement method for determining the fiber orientation direction from the tensile strength ratio in the front-back direction and the width direction.

"Spread state" means a flatly spread state without shrinkage or slackness.

"Stretch rate" means the value when the natural length is taken as 100%.

"Artificial urine" is prepared by mixing urea: 2 wt%, sodium chloride: 0.8 wt%, calcium chloride dihydrate: 0.03 wt%, magnesium sulfate heptahydrate: 0.08 wt%, and ion exchanged water: 97.09 wt%, and those are used at a temperature of 40°C unless otherwise specified.

"Gel strength" is measured as follows: Add 1.0 g of super absorbent polymer to 49.0 g of artificial urine and stir with a stirrer. After leaving generated gel for three hours in a thermo-hygrostat bath at 40°C x 60%RH, return to room temperature, and measure the gel strength with a card meter (Curdmeter-MAX ME-500, manufactured by I. techno Engineering).

"Basis weight" is measured as follows. After preliminary drying a sample or a test piece, the sample or the test piece is left in a test chamber or equipment in the standard state (the test location is at a temperature of 23 ± 1°C and with a relative humidity of 50 ± 2%) to be constant weight. The preliminary drying refers to making a sample or a test piece a constant weight in an environment at a temperature of 100°C. For fibers with an official moisture regain of 0.0%, preliminary drying may not be performed. A sample of dimensions of 100 mm × 100 mm is cut using a template for sampling (100 mm × 100 mm) from a test piece in a constant weight. The basis weight is set by weighing the sample, multiplying by 100, and calculating the weight per one square meter.

"Thickness" is automatically measured under the conditions of a load of 0.098 N/cm$^2$ and a pressing area of 2 cm$^2$ using an automatic thickness measuring device (KES-G5 handy compression testing machine).

"Water absorption" is measured according to JIS K7223-1996 "Test method for water absorption of super absorbent resin".

"Water absorption rate" is the "time to end point" when JIS K7224-1996 "Test method for water absorption rate of super absorbent resin" has been carried out using 2 g of super absorbent polymer and 50 g of physiological saline solution.

When environmental conditions in tests and measurements are not described, the tests and measurements shall be carried out in a test room or apparatus in a standard state (a temperature of 23 ± 1°C and a relative humidity of 50 ± 2% at the test location).

The dimension of each part means the dimension in the spread state, not the natural length state, unless otherwise stated.

Reference Signs List

11      liquid-impervious sheet
12      outer sheet
12T     target sheet
13      fastening tape
13A     engagement portion
13B     tape main unit section
13C     tape attachment part
30      top sheet
40      intermediate sheet
60      three-dimensional side gather
62      gather sheet
50      absorber
51      front surface side sheet
52      back surface side sheet
53      super absorbent polymer particles
54      bonded portion
54b     weak bonded portion
55      cell
80      inner side sheet
H       high shrinkage
L       low shrinkage
LD      front-back direction
WD      width direction

**Claims**

1.  An absorbent article, comprising:

    a front surface side sheet; and
    a back surface side sheet disposed on a back surface side of the front surface side sheet,
    wherein a large number of cells are formed by bonding the front surface side sheet and the back surface side sheet,
    a particulate material containing super absorbent polymer particles is accommodated in the cell to form an absorber,
    at least a part of the particulate material containing the super absorbent polymer particles is not fixed to at least one of the front surface side sheet and the back surface side sheet, and
    in at least one of the front surface side sheet and the back surface side sheet, other sites than the sites where the sheets are bonded each other are shrunk into a pleat shape.

2.  The absorbent article according to claim 1, wherein the pleat shape is a wave shape that contracts in a width direction of the absorber.

3.  The absorbent article according to claim 1 or 2, wherein the pleat shape after shrinking has a shrinkage rate of 15 to 50%.

4.  The absorbent article according to any one of claims 1 to 3, wherein the cell has a rectangular lattice shape that is long in a width direction of the absorber.

5.  The absorbent article according to claim 4, wherein bonded portions extending in a front-back direction among bonded portions forming the rectangular lattice-shaped cells are weak bonded portions.

6.  The absorbent article according to any one of claims 1 to 5, wherein at least one of the front side surface sheet and the back side surface sheet is repeatedly processed into a range in which the shrinkage rate is high and a range in which the shrinkage rate is low.

7.  The absorbent article according to claim 6, wherein a basis weight of the super absorbent polymer particles is higher in a range where the shrinkage rate is high than in a range where the shrinkage rate is low.

## INTERNATIONAL SEARCH REPORT

International application No.

PCT/JP2018/031280

| A. CLASSIFICATION OF SUBJECT MATTER |
|---|
| Int. Cl. A61F13/534(2006.01)i, A61F13/532(2006.01)i |

According to International Patent Classification (IPC) or to both national classification and IPC

| B. FIELDS SEARCHED |
|---|
| Minimum documentation searched (classification system followed by classification symbols) |
| Int. Cl. A61F13/15-13/84, A61L15/16-15/64 |

Documentation searched other than minimum documentation to the extent that such documents are included in the fields searched
Published examined utility model applications of Japan     1922-1996
Published unexamined utility model applications of Japan   1971-2018
Registered utility model specifications of Japan           1996-2018
Published registered utility model applications of Japan    1994-2018

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y<br>A | JP 2011-189067 A (DAIO PAPER CORP.) 29 September 2011, paragraphs [0096]-[0099], [0101], [0108]-[0111], fig. 5, 6, 17, 19-25 (Family: none) | 1-4<br>5-6<br>7 |
| Y | JP 2014-500736 A (VYNKA BVBA) 16 November 2014, paragraphs [0145], [0164], fig. 14 & US 2015/0038929 A1 & EP 2444046 A1, paragraphs [0145], [0164], fig. 14 | 5-6 |
| Y | JP 2013-78369 A (UNI-CHARM CORP.) 02 May 2013, paragraphs [0020], [0032]-[0035], [0037], fig. 8-10 & WO 2013/046702 A1, paragraphs [0018], [0029]-[0032], [0034], fig. 8-10 | 1, 3-5 |

☒ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05.10.2018 | 16.10.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2018/031280

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2014-188105 A (OJI HOLDINGS CORP.) 06 October 2014, paragraph [0053], fig. 4 (Family: none) | 1, 3-5 |
| Y | JP 2001-328191 A (UNI-CHARM CORP.) 27 November 2001, paragraph [0050], fig. 1, 4 & EP 1133962 A1, paragraph [0056], fig. 1A-1B, 4 & KR 10-2001-0091974 A & CN 1313074 A | 1, 3-5 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010063815 A